# EUROPEAN PATENT SPECIFICATION

(11) **EP 2 638 925 B1**
(45) Date of publication and mention of the grant of the patent: **12.04.2017**
(21) Application number: 12159749.6
(22) Date of filing: 15.03.2012
(51) Int. Cl.: A61M 15/00

(54) **Actuator device for inhalers**
Betätigungsvorrichtung für Inhaliergeräte
Dispositif actionneur pour inhalateurs

(43) Date of publication of application: 18.09.2013
(73) Proprietor: Relevo Limited, Aylesbury, Buckinghamshire HP18 9BP (GB)
(72) Inventor: Pinfold, Andrew, Belfast, BT8 8BU (GB)
(74) Representative: Walsh, Marie Goretti

(56) References cited:
- WO-A1-92/07600
- GB-A- 2 266 466
- GB-A- 2 389 316
- US-A- 3 456 644
- US-A- 5 133 343
- US-A- 5 284 133
- US-A1- 2002 100 472
- US-A1- 2004 231 667
- US-A1- 2006 137 681
- US-A1- 2007 240 711
- US-A1- 2011 277 764

## Description

The present invention relates to improved actuator devices for pressurised metered dose inhalers (PMDI's).

An inhaler comprising a housing holding a pressurized canister of atomized medicine for treatment of bronchial conditions such as asthma, is well known. Up until now, the use of such conventional inhalers requires a manual triggering of the inhaler for delivery of the drug through a mouthpiece. Conventional inhalers also rely on manually stored potential energy in a heavy spring to provide energy for the metered delivery of a measured amount of the drug. The ability to provide this function requires the patient to have enough physical strength and movement to prime the inhaler with potential energy and enough strength and movement to trigger the drug delivery. Patients of limited dexterity and limited motor function may have difficulty in achieving these functions.

Document US 2006/0137681 discloses an actuator device for use with metered dose inhalers. This device utilises a breath sensor to trigger the administration of the substance from a canister and an electromagnet to create the force necessary to actuate the canister.

US 2002/0100472 discloses a device for use with metered dose inhalers and includes a housing configured with a void to receive a metered dose inhaler, an actuator assembly which is configured to selectively apply force to the metered dose inhaler to cause the metered dose inhaler to release medicament, and a cocking mechanism for placing the actuator assembly in an armed configuration. The present invention seeks to alleviate the disadvantages associated with the prior art.

The present invention accordingly provides an actuator device as claimed in claim 1 of the appended claims. Preferred features of the actuator device of the invention are set forth in the dependent claims.

The invention provides an actuator device for engagement with an inhaler comprising a housing and a pressurized canister of medicine, the actuator comprising detecting means for detecting the presence of a patient to whom medicine is to be delivered from the pressurized canister; and the actuator comprising an mechanical linear motion to operate a lever which is moveable between an "at rest" position and an "in use" position in which, in use, the lever exerts a force on the canister so as to operate the inhaler and release medicine from the pressurized canister, wherein the means for detecting the presence of a patient comprises a sensor and wherein the sensor comprises a breath activated sensor, and wherein the lever to operate the inhaler is electrically triggered.

Thus, the present invention provides a low voltage electronic means to trigger an electronic timed mechanism to activate a pressurised metred dose inhaler (pMDI) for the patient).

The actuator device of the present invention is a low-pressure triggered actuator device for releasing an atomised drug medicine in the mouth and consequently, in the air passage of a patient requiring delivery of the drug which is contained in the pressurised canister in the inhaler.

The breath activated sensor may comprise a breath flow sensor or a vane sensor.

The actuator device is preferably provided in the form of a single unit for engagement with an inhaler.

The actuator has an electrically triggered lever to operate the inhaler. Preferably, the lever to operate the inhaler is driven by an electrically operated means comprising a servo, a solenoid or a gearbox.

The actuator device of the present invention is adapted for engagement with an inhaler comprising a housing and a pressurised canister. Advantageously, the means for adapting the actuator device for engagement with the inhaler comprise a mouth on the actuator device which is adapted to engage with the inhaler. The mouth of the actuator device ideally includes a seal so that the actuator device can sealingly engage with the inhaler.

Preferably, the actuator device mouth is shaped for clip-in and clip-out engagement with the inhaler so that the actuator device can be easily clipped into and out of engagement with the inhaler.

The actuator device can be clipped onto the mouthpiece of the inhaler. The actuator device can be in the form of a housing which at least partially houses the inhaler when the inhaler is engaged with the actuator device and most preferably, when the inhaler is clipped-into and can be clipped-out of the housing defined by the actuator device. Furthermore, the actuator device ideally comprises a mechanical counter display.

Preferably, the actuator device has a viewing window.

The actuator preferably includes a low power indicator.

The actuator device uses electrically driven mechanical linear motion to trigger the lever to operate the inhaler. Accordingly, the actuator device comprises means for operating the lever comprising a mechanical lever linkage operable to exert a force on the pressurised canister in the inhaler. Advantageously, the force exerted by the lever is a downward force so as to operate the inhaler, releasing medicine from the canister and delivering medicine to the user.

Preferably, the actuator comprises an internal non-volatile memory to facilitate usage recording.

The actuator advantageously comprises an internal timer circuit to control the activation of a lever to operate the inhaler.

The actuator ideally comprises a real time clock for itemised logging of activation.

The actuator ideally comprises an internal data log to record to record the number of times the inhaler has been used.

Optimally, the actuator includes means for downloading data logged in the non-volatile internal memory, said means comprising external interface or Bluetooth for extracting logged data and resetting.

The actuator advantageously has an external accessible battery compartment to enable changing of the battery.

Ideally, the present invention provides a low voltage actuator triggered by sensors so as to correctly operate an inhaler to facilitate patient compliance with appropriate inhaler use.

The actuator allows for battery exchange; and the actuator optimally has twelve months useage.

Preferably, the actuator device also comprises adjustment means for accommodating different sizes of inhaler in the actuator device housing. This adjustment means may comprise an insertable adjusting arm, or, alternatively, the adjustment means may comprise a plurality of insertable seals, each sized seal being insertable in the actuator device mouth such that an appropriate size of seal can be selected from the plurality of insertable seals and can be inserted to correspondingly match the size of the inhaler to be engaged with the actuator device.

This invention provides an actuator device in the form of an easy-grip wrapped plastic shell that serves as a holder for the internal electronics and a battery holder.

The actuator device of the present invention is in the form of a housing into which the inhaler is inserted by clipping in the inhaler. Preferably, the actuator device housing includes an opening defining a mouth and the inhaler is inserted by clipping in the inhaler into the mouth of the actuator device housing. The actuator device mouth includes a seal around the mouth region to sealingly hold the inhaler. Thus the actuator device is in the form of a housing into which an inhaler is inserted and at least partially housed such that the mouthpiece of the inhaler is accessible to the patient for insertion of the inhaler mouthpiece in the patient's mouth for drug delivery. Furthermore, the actuator device comprises a breath activated sensor.

Advantageously, actuator device housing is adapted such that the inhaler is removeably inserted in the housing provided by the actuator device in a clip-in manner. Furthermore, the actuator device housing is adapted such that the inhaler is removable from the housing in an easily operated clip-out involving the user holding a lower portion of the inhaler or underneath the mouthpiece of the inhaler and only a relatively small force is needed to disengage the inhaler from the housing so that the inhaler is removed in a "clip-out" manner.

The features of the actuator device of the present invention are included in the appended claims.

The present invention will now be described more particularly with reference to and as shown in the accompanying drawings, in which are shown, by way of example only, an embodiment of the actuator device of the present invention:

### Figures 1 to 9 relates to the actuatordevice of the present invention:

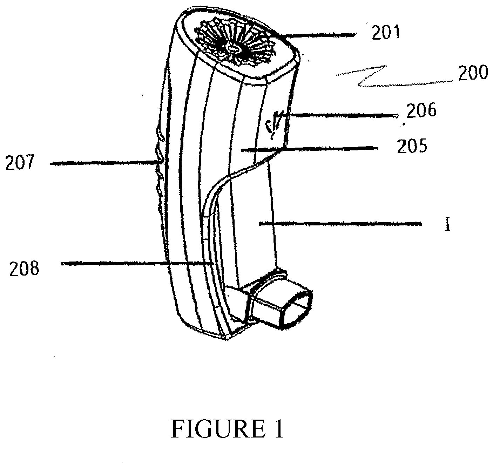

Figure 1 is a perspectiveview of the actuator device engaged with an inhaler;
Figure 1 is a perspectiveview of the actuator device of the present invention engaged with an inhaler, readyfor use;
Figure 2 is a front view of the actuator device, engaged with the inhaler; Figure 3 is a first side viewfrom one side of the actuatordevice of Figure 1;
Figure 4 is a second side viewfrom the other side of the actuator device of Figure 1;
Figure 5a is a plan view from above the actuator device of Figure 1 engaged with an inhaler; Figure 5b is a plan view from underneath of the actuator and inhaler of Figure 1;
Figure 6a is a sectional view showing the internal mechanism for activation of the pressurized canister in the inhaler which is engaged with the actuator device in use;
Figure 6b is an exploded sectional view of the internal mechanism of Figure 6a;
Figure 7 is side view of the actuator device shown without an inhaler inserted in the actuator device;
Figure 8 is a front view of the actuator device of Figure 7;
Figure 9a is a side view of the actuator device as shown in Figure 7 but in partial section, showing an insertable adjuster means for adjusting the size within the housing so as to be capable of engagingly receiving a smaller sized inhaler;
Figure 9b is a front view of the actuator device as shown in Figure 9a;
Figure 9c is a side view of the insertable adjuster means shown by itself, removed from the actuator device of Figure 9a; and
Figure 9d is a front view of the insertable adjuster means shown removed from the actuator device of Figure 9b.

The two embodiments of the actuator device will now be described more particularly with reference to the drawings.

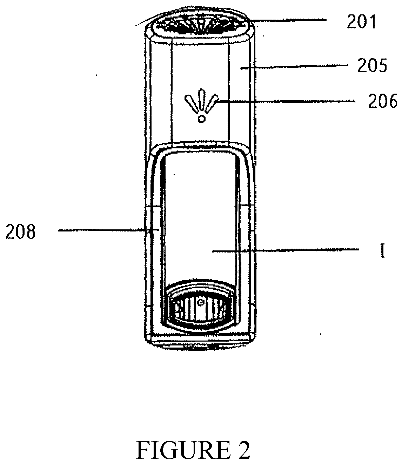

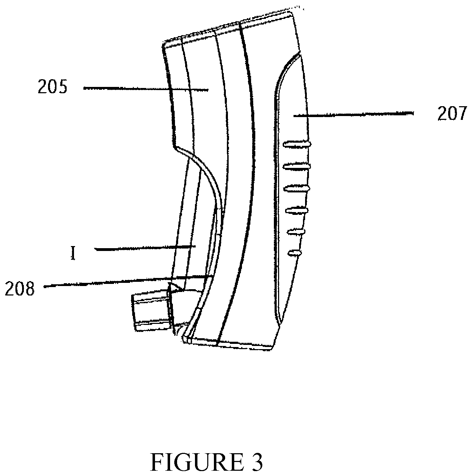

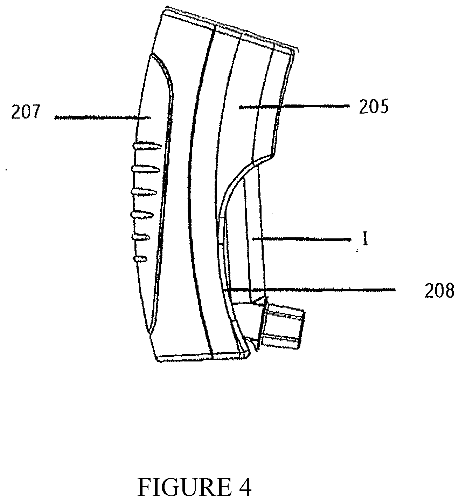

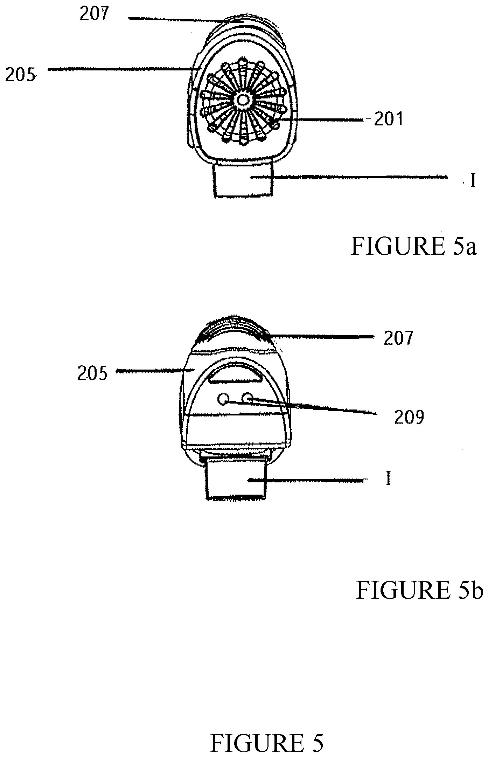

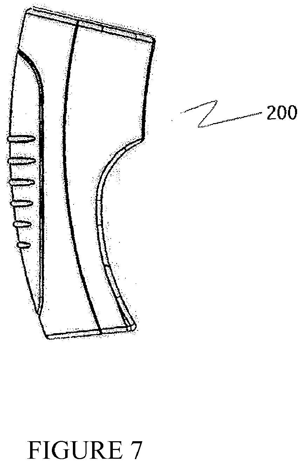

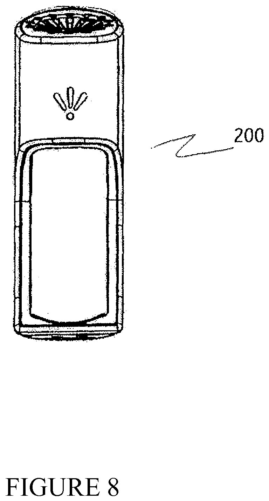

Referring now to Figures 1 to 9 inclusive, the actuator device will now be described. The actuator housing is indicated generally by reference numeral 200.

In Figures 1 to 6, the actuator device 200 is shown ready for use, with an inhaler (comprising an inhaler housing and a pressurised canister) engaged in the actuator device 200.

Referring to Figures 1 to 9d, the actuator 200 includes the following features:
Air vents 201, plastic housing 205, LED light 206, pMDI inhaler I, seal 208, rear hand grip 207 and battery charging points 209.

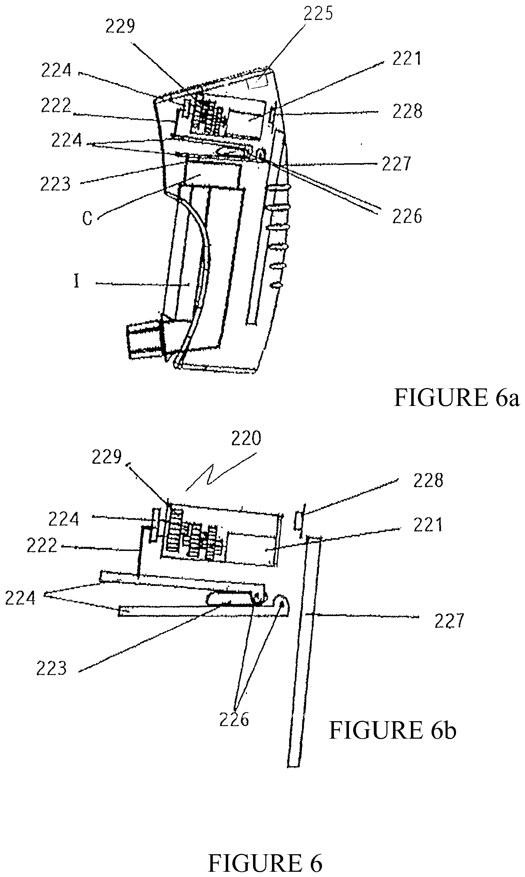

Referring now to Figure 6a and Figure 6b, the sectional view in Figure 6a and exploded sectional view of lever linkage in Figure 6b, the general reference numeral for the mechanism is 220; and the mechanism 220 includes an electrically driven linear motor 221 (e.g. servo, gearbox or solenoid), linkage 222, plastic cam 223; and lever arms 224.

A pMDI canister inhaler A including canister C, lever fixing pins 226, lithium ion battery 227, PCB board 228 and gears 229.

The lithium ion battery 227 lasts up to 1 year. The lithium ion battery 227 may be rechargeable. If it is a rechargeable battery, then the two recharging contact points 209 enable power to flow into the battery when placed in a re-charging unit (not shown in the drawings).

Figures 6a and 6b are section views showing the internal electrical servo gearbox 221, linkage bar 222, lever arms 224, plastic cam 223, pMDI canister C, sensor 225, lever pivoted pins 226, servo or gearbox fixing points 226.

In use, an inhaler is clipped into the housing defined by the actuator device 200 and the mouthpiece of the inhaler will be exposed as shown in Figure 6a. The user positions his/her mouth over the inhaler mouthpiece and inhales. Because of this inhalation, air is drawn in through the air vents 201 and this air flow activates the breath flow sensor 225.

The activation of breath flow sensor 225 or vane sensor, in turn, causes activation of an electrically driven gearbox servo or gearbox 221 which pushes the linkage bar 222 and thereby pushes the lever arms 224 so as to compress the pMDI inhaler canister C to release drug so as to deliver the drug dosage in the optimum way.

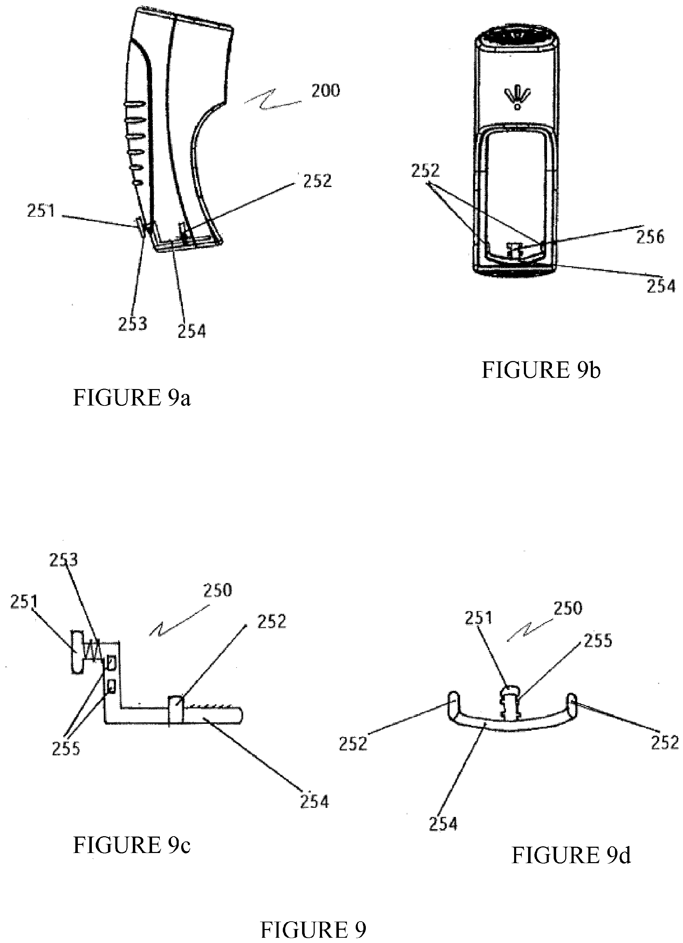

Referring now to Figures 9a to 9d inclusive, an insertable adjusting arm for adjusting the available height within the housing defined by the actuator device 200 so as to accommodate differently sized pMDI inhalers, is shown and is indicated generally by reference numeral 250.

The insertable adjusting arm 250 includes the following features:
Rear push in slide button to adjust height 251, side stabiliser sliding peg 252, button ejecting spring 253, base plate with grips to hold a pMDI inhaler 254, rear locating teeth 255; and rear locating locking points for height adjustment of a pMDI inhaler 256.

In the present invention, the actuator device comprises a breath activated sensor. Furthermore, the actuator device is in the form of a housing into which an inhaler is inserted and at least partially housed such that the mouthpiece of the inhaler is accessible to the patient for insertion of the inhaler mouthpiece in the patient's mouth for drug delivery.

The actuator device housing is adapted such that the inhaler is removeably inserted in the housing provided by the actuator device in a clip-in manner. Furthermore, the actuator device housing is adapted such that the inhaler is removable from the housing in an easily operated clip-out involving the user holding a lower portion of the inhaler or underneath the mouthpiece of the inhaler and only a relatively small force is needed to disengage the inhaler from the housing so that the inhaler is removed in a "clip-out" manner.

Features and advantages of the actuator device 220 include the following:
A portable, battery operated, breath activated pMDI inhaler clip-in attachment is provided by the present invention, for ease of use for patients with reduced or limited dexterity and co-ordination;
electrically driven breath activated clip-in attachment for a pMDI inhaler; clip-in insertion of the inhaler in the actuator device and clip-out withdrawal of the inhaler from the actuator device housing of the actuator device of the present invention; and
clip-on attachment of the actuator device in the first embodiment whereby the actuator device in the first embodiment is engaged with the mouth piece of the inhaler; and
the actuator device in any embodiment of the present invention can engage with most pMDI thereby providing simpler and easier activation and use of any pMDI.

It will of course be understood that aspects of the present invention have been described byway of example only and it should be appreciated that additions and/or modifications may be made thereto without departing form the scope of the present invention as defined in the appended claims.

## Claims

1. An actuator device (200) for engagement with an inhaler (I), the inhaler comprising an inhaler housing holding a pressurized canister of medicine (C) and the inhaler having a mouthpiece; wherein:
- the actuator device (200) is in the form of an actuator housing (205) into which the inhaler (I) can be inserted in a clip-in manner and at least partially housed, such that the mouthpiece of the inhaler (I) is accessible to the patient for insertion of the inhaler mouthpiece in the patient's mouth for delivery of the medicine;
- the actuator housing (205) is configured to extend along the entire length of the inhaler housing on the side opposed the mouthpiece;
- the actuator device (200) comprises a mechanism (220) to operate the inhaler, the mechanism (220) including a lever which is moveable between an "at rest" position and an "in use" position in which, in use, the lever exerts a force on the pressurized canister (C) so as to operate the inhaler (I) and release medicine from the pressurized canister (C), and an electrically driven linear motor (221) to trigger the lever to operate the inhaler (I);
- the actuator device (200) comprises detecting means for detecting the presence of a patient to whom medicine is to be delivered from the pressurized canister (C) and to activate the mechanism (220), wherein the means for detecting the presence of a patient comprises a breath activated sensor (225);
**characterized in that**
- the actuator housing is configured to extend circumferentially about a portion of the inhaler housing, at an end of the inhaler housing opposed the inhaler mouthpiece, to define an enclosed portion of the actuator housing (205),
- the actuator mechanism (220) is housed within the enclosed portion of the actuator housing (205);
- the actuator housing (205) comprises air vents (201) provided on the enclosed portion of the actuator housing (205), whereby in use, when a user positions his/her mouth over the inhaler mouthpiece and inhales, air is drawn in through the air vents (201) and said air flow activates the breath activated sensor (225); and
- the actuator device (200) comprises an internal timer circuit to control the activation of the lever to operate the inhaler (I), the timer circuit providing a delay before delivery of medication from the canister (C) according to the patient's prescription.

2. An actuator device (200) as claimed in claim 1 **characterised in that** the breath activated sensor (225) is provided adjacent to the air vents (201).

3. An actuator device (200) as claimed in any one of claims 1 or 2, wherein the mechanism (220) comprises a linkage (222), a plastic cam (223) and lever arms (224).

4. An actuator device (200) as claimed in any one of claims 1 to 3, having a second sensor for detecting inhalation and/or exhalation to facilitate patient compliance of inhaler use with recommended drug delivery dosage to the patient.

5. An actuator device (200) as claimed in any one of the preceding claims having a clip-in connection for engaging the actuator device with the inhaler (I) wherein the inhaler is clipped-into the housing (205) defined by the actuator device.

6. An actuator device (200) according to any one of the preceding claims wherein the electrically driven linear motor (221) comprises a servo, a solenoid or a gearbox.

7. An actuator device (200) according to any one of the preceding claims having a mechanical counter display; optionally the actuator device having a viewing window.

8. An actuator device (200) as claimed in any one of the preceding claims further comprising a mouth on the actuator device which is adapted to engage with the inhaler (I), the mouth including a seal so that the actuator device (200) can sealingly engage with the inhaler (I).

9. An actuator device (200) as claimed in any one of the preceding claims, wherein the actuator device (200) further comprises an internal non-volatile memory to facilitate usage recording.

10. An actuator device (200) as claimed in any one of the preceding claims, wherein the actuator device (200) further comprises a real time clock for itemised logging of activation.

11. An actuator device (200) as claimed in any one of the preceding claims, wherein the actuator device (200) comprises an internal data log to record the number of times the inhaler (I) has been used.

12. An actuator device (200) as claimed in claim 11, wherein the actuator device (200) comprises means for downloading data logged in the non-volatile internal memory, said means comprising an external interface or Bluetooth for extracting logged data and resetting.

13. An actuator device (200) as claimed in any one of the preceding claims, wherein the actuator is a low voltage actuator triggered by the sensor (225) so as to correctly operate the inhaler (I) to facilitate patient compliance with appropriate inhaler use.

14. An actuator device (200) as claimed in claim 13, wherein the actuator includes a low power indicator.

15. An actuator device (200) as claimed in any one of the preceding claims including means for enabling battery exchange.

16. An actuator device (200) as claimed in claim 15, wherein the actuator device (200) comprises an external accessible battery compartment to enable changing of the battery; and optionally wherein the battery (207) provides twelve months usage.

17. An actuator device (200) as claimed in any preceding claim wherein the actuator device (200) also comprises adjustment means (250) for accommodating different sizes of inhalers (I) in the actuator device housing (205); optionally wherein said adjustment means (250) comprises an insertable adjusting arm; or optionally wherein the adjustment means (250) comprises a plurality of seals insertable in the actuator device mouth such that an appropriate size of seal (208) can be inserted to correspondingly match the size of the inhaler (I) to be engaged with the actuator device (200).

## Patentansprüche

1. Betätigungsvorrichtung (200) zum Ineingrifftreten mit einem Inhaliergerät (I), wobei das Inhaliergerät ein Inhaliergerätgehäuse umfasst, das einen unter Druck stehenden Behälter für Medizin (C) umfasst, und wobei das Inhaliergerät ein Mundstück aufweist; wobei:
- die Betätigungsvorrichtung (200) die Form eines Betätigergehäuses (205) hat, in welches das Inhaliergerät (I) auf eine einrastende Weise und wenigstens teilweise eingehaust eingeführt werden kann, so dass das Mundstück des Inhaliergeräts (I) dem Patienten zur Einführung des Inhaliergerätmundstücks in den Mund des Patienten zur Zuführung der Medizin zugänglich ist;
- das Betätigergehäuse (205) konfiguriert ist, um sich auf der dem Mundstück gegenüberliegenden Seite entlang der gesamten Länge des Inhaliergerätgehäuses zu erstrecken;
- die Betätigungsvorrichtung (200) einen Mechanismus (220) umfasst, um das Inhaliergerät zu betreiben, wobei der Mechanismus (220) einen Hebel beinhaltet, der bewegbar ist zwischen einer Position "im Ruhezustand" und einer Position "im Gebrauch", in welcher der Hebel im Gebrauch eine Kraft auf den unter Druck stehenden Behälter (C) ausübt, um das Inhaliergerät (I) zu betreiben und Medizin aus dem unter Druck stehenden Behälter (C) freizusetzen, und einen elektrisch angetriebenen linearen Motor (221), um den Hebel auszulösen, um das Inhaliergerät (I) zu betreiben;
- die Betätigungsvorrichtung (200) ein Erkennungsmittel zum Erkennen der Präsenz eines Patienten umfasst, dem die Medizin aus dem unter Druck stehenden Behälter (C) zuzuführen ist, und den Mechanismus (220) zu aktivieren, wobei das Mittel zum Erkennen der Präsenz eines Patienten einen atemaktivierten Sensor (225) umfasst;
**dadurch gekennzeichnet, dass**
- das Betätigergehäuse konfiguriert ist, um sich an einem dem Inhaliergerätmundstück gegenüberliegenden Ende des Inhaliergerätgehäuses umlaufend um einen Abschnitt des Inhaliergerätgehäuses zu erstrecken, um einen eingefassten Abschnitt des Betätigergehäuses (205) zu definieren,
- der Betätigermechanismus (220) innerhalb des eingefassten Abschnitts des Betätigergehäuses (205) eingefasst ist;
- das Betätigergehäuse (205) Luftschlitze (201) auf dem eingefassten Abschnitt des Betätigergehäuses (205) umfasst, wobei, wenn ein Benutzer/eine Benutzerin im Gebrauch seinen/ihren Mund über das Inhaliergerätmundstück positioniert und einatmet, Luft durch die Luftschlitze (201) eingezogen wird und der Luftstrom den atemaktivierten Sensor (225) aktiviert; und
- die Betätigungsvorrichtung (200) eine innere Zeitschaltung umfasst, um die Aktivierung des Hebels zu steuern, um das Inhaliergerät (I) zu betreiben, wobei die Zeitschaltung eine Verzögerung vor der Zuführung von Medizin aus dem Behälter (C) gemäß der Verordnung des Patienten bereitstellt.

2. Betätigungsvorrichtung (200) nach Anspruch 1, **dadurch gekennzeichnet, dass** der atemaktivierte Sensor (225) den Luftschlitzen (201) benachbart bereitgestellt ist.

3. Betätigungsvorrichtung (200) nach einem der Ansprüche 1 oder 2, wobei der Mechanismus (220) eine Verknüpfung (222), eine Kunststoffnocke (223) und Hebelarme (224) umfasst.

4. Betätigungsvorrichtung (200) nach einem der Ansprüche 1 bis 3, die einen zweiten Sensor zum Erkennen von Einatmen und/oder Ausatmen aufweist, um die Einhaltung des Inhaliergerätgebrauchs mit der empfohlenen Arzneizuführungsdosierung des Patienten durch den Patienten zu vereinfachen.

5. Betätigungsvorrichtung (200) nach einem der vorhergehenden Ansprüche mit einer Einrastverbindung zum Ineingrifftreten der Betätigungsvorrichtung mit dem Inhaliergerät (I), wobei das Inhaliergerät in das von der Betätigungsvorrichtung definierte Gehäuse (205) eingerastet wird.

6. Betätigungsvorrichtung (200) nach einem der vorhergehenden Ansprüche, wobei der elektrisch angetriebene lineare Motor (221) einen Servomechanismus, ein Solenoid oder ein Getriebe umfasst.

7. Betätigungsvorrichtung (200) nach einem der vorhergehenden Ansprüche mit einer mechanischen Zähleranzeige; wobei der Betätiger gegebenenfalls ein Sichtfenster aufweist.

8. Betätigungsvorrichtung (200) nach einem der vorhergehenden Ansprüche, weiter umfassend einen Mund auf der Betätigungsvorrichtung, der dafür ausgelegt ist, mit dem Inhaliergerät (I) in Eingriff zu treten, wobei der Mund eine Dichtung beinhaltet, so dass die Betätigungsvorrichtung (200) abdichtend mit dem Inhaliergerät (I) in Eingriff treten kann.

9. Betätigungsvorrichtung (200) nach einem der vorhergehenden Ansprüche, wobei die Betätigungsvorrichtung (200) weiter einen internen, nichtflüchtigen Speicher umfasst, um Gebrauchsaufzeichnung zu vereinfachen.

10. Betätigungsvorrichtung (200) nach einem der vorhergehenden Ansprüche, wobei die Betätigungsvorrichtung (200) weiter eine Echtzeituhr zur Einzelerfassung von Aktivierung umfasst.

11. Betätigungsvorrichtung (200) nach einem der vorhergehenden Ansprüche, wobei die Betätigungsvorrichtung (200) eine interne Datenerfassung umfasst, um die Anzahl von Malen aufzuzeichnen, die das Inhaliergerät (I) gebraucht wurde.

12. Betätigungsvorrichtung (200) nach Anspruch 11, wobei die Betätigungsvorrichtung (200) Mittel zum Herunterladen von in dem nichtflüchtigen internen Speicher erfassten Daten umfasst, wobei die Mittel eine externe Schnittstelle oder Bluetooth zum Extrahieren erfasster Daten und Zurücksetzen umfassen.

13. Betätigungsvorrichtung (200) nach einem der vorhergehenden Ansprüche, wobei der Betätiger ein Niedrigspannungsbetätiger ist, der von dem Sensor (225) ausgelöst wird, um das Inhaliergerät (I) richtig zu betreiben, um die Einhaltung des geeigneten Inhaliergerätgebrauchs durch den Patienten zu vereinfachen.

14. Betätigungsvorrichtung (200) nach Anspruch 13, wobei der Betätiger einen Niedrigstromindikator beinhaltet.

15. Betätigungsvorrichtung (200) nach einem der vorhergehenden Ansprüche, die Mittel zum Ermöglichen von Batterieaustausch beinhaltet.

16. Betätigungsvorrichtung (200) nach Anspruch 15, wobei die Betätigungsvorrichtung (200) ein extern zugängliches Batteriefach umfasst, um den Austausch der Batterie zu ermöglichen; und wobei die Batterie (207) gegebenenfalls zwölf Monate Verwendung bereitstellt.

17. Betätigungsvorrichtung (200) nach einem der vorhergehenden Ansprüche, wobei die Betätigungsvorrichtung (200) auch Anpassungsmittel (250) umfasst, um verschiedene Größen von Inhaliergeräten (I) in dem Betätigungsvorrichtungsgehäuse (205) unterzubringen; wobei das Anpassungsmittel (250) gegebenenfalls einen einführbaren Anpassungsarm umfasst; oder wobei das Anpassungsmittel (250) gegebenenfalls eine Vielzahl von Dichtungen umfasst, die in den Betätigungsvorrichtungsmund einführbar sind, so dass eine geeignete Größe der Dichtung (208) eingeführt werden kann, um entsprechend zur Größe des mit der Betätigungsvorrichtung (200) in Eingriff zu bringenden Inhaliergeräts (I) zu passen.

## Revendications

1. Dispositif actionneur (200) pour une mise en prise avec un inhalateur (I), l'inhalateur comprenant un boîtier d'inhalateur contenant une cartouche sous pression de médicament (C) et l'inhalateur ayant un embout ; dans lequel :
- le dispositif actionneur (200) se présente sous la forme d'un boîtier d'actionneur (205) dans lequel l'inhalateur (I) peut être inséré de manière clipsable et peut être au moins partiellement logé, de sorte que l'embout de l'inhalateur (I) soit accessible au patient pour l'insertion de l'embout de l'inhalateur dans la bouche du patient pour la délivrance du médicament ;
- le boîtier d'actionneur (205) est configuré pour s'étendre sur la longueur entière du boîtier d'inhalateur sur le côté opposé à l'embout ;
- le dispositif actionneur (200) comprend un mécanisme (220) pour faire fonctionner l'inhalateur, le mécanisme (220) comportant un levier qui est mobile entre une position « au repos » et une position « en utilisation » dans laquelle, en utilisation, le levier exerce une force sur la cartouche sous pression (C) de façon à faire fonctionner l'inhalateur (I) et à libérer le médicament de la cartouche sous pression (C), et un moteur linéaire entraîné électriquement (221) pour déclencher le levier afin de faire fonctionner l'inhalateur (I) ;
- le dispositif actionneur (200) comprend un moyen de détection pour détecter la présence d'un patient auquel le médicament doit être délivré à partir de la cartouche sous pression (C) et pour activer le mécanisme (220), dans lequel le moyen pour détecter la présence d'un patient comprend un capteur activé par la respiration (225) ;
**caractérisé en ce que**
- le boîtier d'actionneur est configuré pour s'étendre circonférentiellement autour d'une portion du boîtier d'inhalateur, au niveau d'une extrémité du boîtier d'inhalateur opposée à l'embout de l'inhalateur, afin de définir une portion enfermée du boîtier d'actionneur (205),
- le mécanisme actionneur (220) est logé à l'intérieur de la portion enfermée du boîtier d'actionneur (205) ;
- le boîtier d'actionneur (205) comprend des ouvertures d'aération (201) prévues sur la portion enfermée du boîtier d'actionneur (205), moyennant quoi en utilisation, lorsqu'un utilisateur/une utilisatrice positionne sa bouche sur l'embout de l'inhalateur et inhale, de l'air est aspiré par les ouvertures d'aération (201) et ledit écoulement d'air active le capteur activé par la respiration (225) ; et
- le dispositif actionneur (200) comprend un circuit de minuterie interne pour contrôler l'activation du levier afin de faire fonctionner l'inhalateur (I), le circuit de minuterie assurant un délai avant la délivrance du médicament à partir de la cartouche (C) selon la prescription du patient.

2. Dispositif actionneur (200) selon la revendication 1, **caractérisé en ce que** le capteur activé par la respiration (225) est prévu adjacent aux ouvertures d'aération (201).

3. Dispositif actionneur (200) selon l'une quelconque des revendications 1 et 2, dans lequel le mécanisme (220) comprend une articulation (222), une came plastique (223) et des bras de levier (224).

4. Dispositif actionneur (200) selon l'une quelconque des revendications 1 à 3, comportant un second capteur pour détecter une inhalation et/ou une expiration afin de faciliter l'observance du patient à l'utilisation de l'inhalateur avec la posologie de délivrance médicamenteuse recommandée au patient.

5. Dispositif actionneur (200) selon l'une quelconque des revendications précédentes, ayant un raccordement clipsable pour mettre en prise le dispositif actionneur avec l'inhalateur (I), dans lequel l'inhalateur est clipsé dans le boîtier (205) défini par le dispositif actionneur.

6. Dispositif actionneur (200) selon l'une quelconque des revendications précédentes, dans lequel le moteur linéaire entraîné électriquement (221) comprend un servomécanisme, un solénoïde ou une boîte à engrenages.

7. Dispositif actionneur (200) selon l'une quelconque des revendications précédentes, ayant un affichage de compteur mécanique ; facultativement le dispositif actionneur comportant une fenêtre de visualisation.

8. Dispositif actionneur (200) selon l'une quelconque des revendications précédentes, comprenant en outre une entrée sur le dispositif actionneur qui est adaptée pour se mettre en prise avec l'inhalateur (I), l'entrée comportant un joint de sorte que le dispositif actionneur (200) puisse se mettre en prise à étanchéité avec l'inhalateur (I).

9. Dispositif actionneur (200) selon l'une quelconque des revendications précédentes, dans lequel le dispositif actionneur (200) comprend en outre une mémoire non volatile interne afin de faciliter l'enregistrement de l'emploi.

10. Dispositif actionneur (200) selon l'une quelconque des revendications précédentes, dans lequel le dispositif actionneur (200) comprend en outre une horloge en temps réel pour la journalisation détaillée de l'activation.

11. Dispositif actionneur (200) selon l'une quelconque des revendications précédentes, dans lequel le dispositif actionneur (200) comprend un journal de données interne afin d'enregistrer le nombre de fois où l'inhalateur (I) a été utilisé.

12. Dispositif actionneur (200) selon la revendication 11, dans lequel le dispositif actionneur (200) comprend un moyen pour télécharger des données journalisées dans la mémoire interne non volatile, ledit moyen comprenant une interface externe ou un Bluetooth pour l'extraction de données journalisées et la réinitialisation.

13. Dispositif actionneur (200) selon l'une quelconque des revendications précédentes, dans lequel l'actionneur est un actionneur basse tension déclenché par le capteur (225), de sorte à faire fonctionner de façon correcte l'inhalateur (I) afin de faciliter l'observance du patient avec l'utilisation appropriée de l'inhalateur.

14. Dispositif actionneur (200) selon la revendication 13, dans lequel l'actionneur comporte un indicateur de faible puissance.

15. Dispositif actionneur (200) selon l'une quelconque des revendications précédentes, comportant un moyen pour permettre un échange de batterie.

16. Dispositif actionneur (200) selon la revendication 15, dans lequel le dispositif actionneur (200) comprend un compartiment de batterie accessible externe afin de permettre le changement de la batterie ; et facultativement dans lequel la batterie (207) permet un emploi de douze mois.

17. Dispositif actionneur (200) selon une quelconque revendication précédente, dans lequel le dispositif actionneur (200) comprend également un moyen d'ajustement (250) pour recevoir différentes tailles d'inhalateurs (I) dans le boîtier de dispositif actionneur (205) ; facultativement dans lequel ledit moyen d'ajustement (250) comprend un bras d'ajustement insérable ; ou facultativement dans lequel le moyen d'ajustement (250) comprend une pluralité de joints insérables dans l'entrée de dispositif actionneur, de sorte qu'une taille appropriée de joint (208) puisse être insérée pour s'apparier en correspondance à la taille de l'inhalateur (I) à mettre en prise avec le dispositif actionneur (200).
